# EUROPÄISCHE PATENTANMELDUNG

(11) **EP 3 659 640 A1**
(43) Veröffentlichungstag der Anmeldung: **03.06.2020**
(21) Anmeldenummer: 18208492.1
(22) Anmeldetag: 27.11.2018
(51) Int. Cl.: A61M 1/10, A61M 1/12

(54) **VERFAHREN ZUR HERSTELLUNG EINER LAGERANORDNUNG FÜR EINE IMPLANTIERBARE BLUTPUMPE, LAGERANORDNUNG UND IMPLANTIERBARE BLUTPUMPE**

(71) Anmelder: Berlin Heart GmbH, 12247 Berlin (DE)
(72) Erfinder: Richert, Hendryk, 12487 Berlin (DE); Peters, Oliver, 14129 Berlin (DE)
(74) Vertreter: Pfenning, Meinig & Partner mbB

(57) **Zusammenfassung**

Die vorliegende Anmeldung betrifft ein Verfahren zur Herstellung einer Lageranordnung für eine implantierbare Blutpumpe (2), eine Lageranordnung und eine implantierbare Blutpumpe (2). Bei dem vorgeschlagenen Verfahren wird ein Rotor (8) mit einem oder mehreren Antriebsmagneten bereitgestellt. Der Rotor (8) weist ein Förderelement (9) auf. Außerdem wird ein Stator (13) mit Statorwindungen bereitgestellt. Des Weiteren wird der Rotor (8) in einem durch eine Innenwandung des Stators (13) gebildeten Strömungskanal (10) angeordnet. Anschließend wird eine Rotordrehung angetrieben. Während die Rotordrehung angetrieben wird, wird eine Auslenkung des Rotors (8) bestimmt. In einem weiteren Schritt wird die Auslenkung des Rotors (8) durch nicht rotationssymmetrisches Aufbringen, Entfernen, Magnetisieren und/oder Entmagnetisieren von magnetisch wirksamem Material an dem Stator (13) und/oder an dem Rotor (8) korrigiert.

## Beschreibung

Die vorliegende Anmeldung liegt auf dem Gebiet der Medizintechnik und insbesondere auf dem Gebiet implantierbarer Blutpumpen zur Unterstützung einer Herzfunktion. Die Anmeldung betrifft ein Verfahren zur Herstellung einer Lageranordnung für eine implantierbare Blutpumpe, eine Lageranordnung und eine implantierbare Blutpumpe.

Blutpumpen sind aus dem Stand der Technik bekannt. Diese Blutpumpen können zum Einsatz kommen, wenn eine Herzfunktion eines Patienten unterstützt oder ersetzt werden muss. Gängige Systeme, die hierbei verwendet werden, sind sogenannte VAD (ventricular assist devices). Derartige Herzpumpen können beispielsweise als sog. LVAD (left ventricular assist device), RVAD (right ventricular assist device) oder BiVAD (bi-ventricular assist device) ausgeführt sein. Diese Systeme umfassen neben der Blutpumpe, die im Betrieb in dem Patienten implantiert ist, in der Regel ein beispielsweise außerhalb eines Körpers des Patienten angeordnetes und mit der Blutpumpe über eine Leitung (Driveline) verbundenes Steuergerät. Die Blutpumpe umfasst in der Regel einen Motor mit einem Stator und einem mit einer Beschaufelung versehenen Rotor, der in einem Strömungskanal der Blutpumpe angeordnet ist. Durch von dem Steuergerät gelieferte Energie kann der Motor der Blutpumpe angetrieben werden, indem beispielsweise ein Stromfluss in Wicklungen des Stators erzeugt wird, durch den der Rotor mitsamt Beschaufelung zum Fördern von Blut des Patienten in Drehung versetzt wird.

Insbesondere bei magnetisch gelagerten Blutpumpen, die eine vergleichsweise geringe Spaltbreite zwischen der Beschaufelung des Rotors und einer Innenwand des Strömungskanals aufweisen, was insbesondere für axiale und halbaxiale Strömungsgeometrien zutrifft, kann eine genau kontrollierbare Lage des Rotors im Strömungskanal der Blutpumpe notwendig sein für eine sichere Funktion der Blutpumpe. Beispielsweise beschreibt die Druckschrift EP 3 300 749 A1 eine Blutpumpe mit einem Rotor, der radial passiv magnetisch gelagert ist. Wenn eine präzise Einstellung der Rotorlage notwendig ist, kann eine Herstellung des Magnetlagers der Blutpumpe vergleichsweise aufwendig sein, da als Ausgangsmaterial für das Magnetlager verwendete Permanentmagneten in der Regel eine nicht zu vernachlässigende Toleranz in ihren magnetischen Eigenschaften aufweisen können. Zur Herstellung einer Blutpumpe mit präzise eingestellter Rotorlage können beispielsweise mehrere Permanentmagneten in der Blutpumpe eingebaut, hinsichtlich ihrer magnetischen Eigenschaften getestet und solange ausgetauscht werden, bis die gewünschten magnetischen Eigenschaften vorliegen. Ein solches Verfahren ist jedoch vergleichsweise aufwendig und wird zu vermehrtem Ausschuss führen.

Es ist eine Aufgabe der vorliegenden Anmeldung ein verbessertes Verfahren zur Herstellung einer Lageranordnung für eine implantierbare Blutpumpe vorzuschlagen. Insbesondere soll das vorgeschlagene Verfahren vergleichsweise schnell und kostengünstig sein und eine Herstellung einer Blutpumpe ermöglichen, bei der die magnetischen Eigenschaften und die Rotorlage präzise eingestellt sind. Außerdem ist es eine Aufgabe der vorliegenden Anmeldung eine entsprechend vorteilhafte Lageranordnung sowie eine entsprechend vorteilhafte implantierbare Blutpumpe vorzuschlagen.

Diese Aufgaben werden gelöst durch ein Verfahren mit den Merkmalen des unabhängigen Anspruchs 1 und durch eine Lageranordnung bzw. eine Blutpumpe mit den Merkmalen eines weiteren Anspruchs. Vorteilhafte Weiterbildungen ergeben sich mit den Merkmalen der abhängigen Ansprüche und der Ausführungsbeispiele.

Bei dem vorgeschlagenen Verfahren zur Herstellung einer Lageranordnung für eine implantierbare Blutpumpe wird ein Rotor mit einem oder mehreren Antriebsmagneten bereitgestellt. Der Rotor weist ein Förderelement auf. Außerdem wird ein Stator mit Statorwindungen bereitgestellt. Des Weiteren wird der Rotor in einem durch eine Innenwandung des Stators gebildeten Strömungskanal angeordnet. Durch den Strömungskanal wird Blut gefördert, wenn die Blutpumpe implantiert ist und betrieben wird. Dabei wird eine Rotordrehung, insbesondere durch Erzeugung eines Stromflusses in den Statorwindungen, angetrieben. Während des Herstellungsprozesses wird die Rotordrehung angetrieben wird, und es wird dabei eine Auslenkung des Rotors bestimmt. In einem weiteren Schritt wird die Auslenkung des Rotors durch nicht rotationssymmetrisches Aufbringen, Entfernen, Magnetisieren und/oder Entmagnetisieren von magnetisch wirksamem Material an dem Stator und/oder an dem Rotor korrigiert. Die Lageranordnung kann in einigen Ausführungen Teil eines Elektromotors der Blutpumpe sein, der außerdem den Stator und den Rotor umfasst.

Durch das vorgeschlagene Verfahren können Lageranordnungen für Blutpumpen hergestellt werden, die eine präzise Positionierung des Rotors im Betrieb ermöglichen. Dadurch weisen diese Lageranordnungen eine große Laufruhe auf und sind dadurch langlebig sowie energieeffizient. Außerdem kann die Pumpe in einem breiteren Arbeitsbereich betrieben werden, wenn der Rotor in seiner Neutrallage exakt positionierbar ist. Durch die Verfahrensschritte der Bestimmung der Auslenkung des Rotors lässt sich eine gegebenenfalls vorliegende und durch die verwendeten Stator- und Rotormagneten hervorgerufene magnetische Unwucht feststellen und anschließende gezielt korrigieren. Unter der Bestimmung der Auslenkung des Rotors ist die Bestimmung einer Position oder eines Abstands oder eines Winkels des Rotors relativ zu einem Bezugspunkt oder einer Bezugsachse zu verstehen. Durch diesen Schritt wird gegenüber Herstellungsverfahren, bei denen eine Vielzahl unterschiedlicher Rotor- bzw. Statormagnete getestet und selektiert werden, erreicht, dass Blutpumpen mit den gewünschten magnetischen Eigenschaften bei weniger Ausschuss und somit materialsparend und kostengünstig herstellbar sind. Ein weiterer Vorteil ist, dass die nach dem vorgeschlagenen Verfahren magnetisch korrigierten Rotoren und Statoren bei ihrem endgültigen Zusammenbau in der Regel keine weiteren Maßnahmen zur Korrektur der magnetischen Eigenschaften benötigen, so dass nachfolgende Schritte bei der Herstellung des Elektromotors bzw. der Blutpumpe vergleichsweise schnell und einfach durchführbar sind. Die vorliegende Anmeldung kann darüber hinaus ein Verfahren zur Herstellung der Blutpumpe betreffen, das die Verfahrensschritte zur Herstellung der Lageranordnung umfasst.

Es ist in bevorzugten Ausführungen vorgesehen, dass die Lageranordnung ein magnetisches Radiallager mit mindestens einem Rotorlagermagneten und mindestens einem Statorlagermagneten umfasst. Die Auslenkung des Rotors kann eine radiale Auslenkung sein. Es kann zudem vorgesehen sein, dass während des Antriebs der Rotordrehung die radiale Auslenkung des Rotors bestimmt wird. Anschließend kann die radiale Auslenkung wie oben oder unten beschrieben korrigiert werden. Hierbei wird die radiale Auslenkung, beispielsweise in Bezug auf einen Mittelpunkt bzw. eine Zylindersymmetrieachse des Strömungskanals, in der Regel reduziert. Auf diese Weise wird eine genaue Kontrolle über die Spaltbreite im Strömungskanal zwischen Stator und Rotor ermöglicht.

Die Rotationseigenschaften des Rotors sind abhängig von einer Drehzahl, bei der der Elektromotor betrieben wird. Der Rotor kann hierbei bei einer unterkritischen (geringen) Drehzahl (unterhalb der Resonanzfrequenz) um seine magnetische Schwerpunktachse rotieren und eine Taumelbewegung vollführen und bei einer überkritischen (hohen) Drehzahl (oberhalb der Resonanzfrequenz) um seine Massenträgheitsachse rotieren.

In einigen Ausführungen wird die Bestimmung der radialen Auslenkung bei einem Antrieb der Rotordrehung mit einer Drehzahl durchgeführt, bei der der Rotor im Wesentlichen um seine Massenträgheitsachse rotiert. Anschließend kann die radiale Auslenkung des Rotors durch nicht rotationssymmetrisches Aufbringen, Entfernen, Magnetisieren und/oder Entmagnetisieren von magnetisch wirksamem Material an dem Stator reduziert werden.

Der Bezugspunkt zur Bestimmung der Auslenkung kann hierbei beispielsweise ein Mittelpunkt bzw. eine Zylindersymmetrieachse des Strömungskanals sein. Nach der Korrektur kann eine geometrische Schwerpunktachse des Rotors beispielsweise mit der Zylindersymmetrieachse des Strömungskanals übereinstimmen, so dass der Rotor in einem Arbeitsbereich im Wesentlichen mittig im Strömungskanal liegt bzw. rotiert.

Hierbei wird die Bestimmung der Auslenkung bei vergleichsweise hoher Drehzahl durchgeführt, bei der der Rotor selbststabilisiert um seine Massenträgheitsachse rotiert. Die Lage des Rotors ist hierbei normalerweise stabil, so dass die Auslenkung zeitunabhängig ist. Die Lage des Rotors wird hierbei in der Regel hauptsächlich durch das Magnetfeld des Statorlagermagneten beeinflusst. Somit kann durch die Korrektur am Stator eine präzise Einstellung der Rotorlage erreicht werden. Typischerweise wird der Rotor bei der Bestimmung seiner Auslenkung im überkritischen Zustand betrieben. Die Resonanzdrehzahl, oberhalb welcher der Rotor überkritisch betrieben wird, ist in der Regel geometrieabhängig und kann beispielsweise zumindest 2000 rpm betragen, insbesondere bei radialen Pumpensystemen mehr als 3000 rpm und insbesondere bei axialen Pumpensystemen mehr als 6000 rpm.

Es kann zusätzlich oder alternativ vorgesehen sein, dass die Bestimmung der radialen Auslenkung bei einem Antrieb der Rotordrehung mit einer Drehzahl durchgeführt wird, bei der der Rotor im Wesentlichen um seine magnetische Schwerpunktachse rotiert. Anschließend kann die Auslenkung des Rotors durch nicht rotationssymmetrisches Aufbringen, Entfernen, Magnetisieren und/oder Entmagnetisieren von magnetisch wirksamem Material an dem Rotor korrigiert werden.

Hierbei erfolgt die Bestimmung der Auslenkung des Rotors in der Regel bei geringen Drehzahlen, bei der der Rotor um seinen magnetischen Schwerpunkt rotiert und bei der die Auslenkung des Rotors daher zeitabhängig ist, sodass der Rotor eine Art Taumel- oder Schwingungsbewegung durchführt. Hierbei kann durch eine Korrektur der magnetischen Unwucht am Rotor besonders effizient eine Kontrolle über die Rotorlage erreicht werden. Die Bestimmung der Auslenkung erfolgt dann in der Regel im unterkritischen Betrieb bei Drehzahlen von höchstens 500 Umdrehungen pro Minute. Die Korrektur der magnetischen Unwucht am Rotor wird in der Regel so durchgeführt, dass der Rotor anschließend stabil bei zeitlich konstanter radialer Auslenkung rotiert.

In einigen Ausführungen erfolgt für eine präzise Einstellung der magnetischen Eigenschaften der Lageranordnung bzw. des Elektromotors zunächst die Korrektur bei unterkritischen Bedingungen und anschließend die Korrektur bei überkritischen Bedingungen.

Es kann beispielsweise in einigen Ausführungen vorgesehen sein, dass beim Antrieb der Rotordrehung eine Flüssigkeit im Strömungskanal zwischen Stator und Rotor angeordnet ist. Somit erfolgt die Messung annähernd unter realen Betriebsbedingungen, bei denen Blut im Strömungskanal angeordnet ist und durch diesen gefördert wird.

Die Auslenkung des Rotors während des Antriebs der Rotordrehung kann beispielsweise in besonders einfacher Weise mittels Mikroskop und/oder mittels Abstandssensoren und/oder mittels Beschleunigungssensoren bestimmt werden.

In typischen Ausführungen wird die Auslenkung des Rotors durch Aufbringen eines magnetisierbaren und/oder magnetisierten Materials korrigiert. Dieses Material kann insbesondere ein permanentmagnetisches oder weichmagnetisches Material, zum Beispiel NdFeB oder Eisenblech oder eine weichmagnetische Legierung, sein. Dadurch kann die Korrektur vergleichsweise einfach, vorhersehbar und besonders zielführend durchgeführt werden. Dies gilt insbesondere dann, wenn die Korrektur am Stator durchgeführt wird, da in diesem Fall gegebenenfalls bestehende oder erzeugte mechanische Unwuchten nicht zu berücksichtigen sind.

Es kann beispielsweise vorgesehen sein, dass die Korrektur der Auslenkung des Rotors durch nicht rotationssymmetrisches Aufbringen oder Entfernen von magnetisch wirksamem Material an dem Rotor durchgeführt wird. Vorteilhafterweise kann in diesem Fall zusätzlich magnetisch nicht wirksames Material, zum Beispiel unmagnetischer Edelstahl oder Messing, zum Korrigieren einer mechanischen Unwucht des Rotors vom Rotor entfernt bzw. auf den Rotor aufgebracht werden.

Die mechanische Unwucht kann beispielsweise durch das magnetische Korrigieren der Auslenkung des Rotors hervorgerufen sein. Beispielsweise kann das magnetisch nicht wirksame Material derart vom Rotor entfernt bzw. auf den Rotor aufgebacht werden, dass ein Einfluss des magnetischen Korrigierens auf die Massenträgheitsachse des Rotors ausgeglichen wird. Somit kann auf diese Weise erreicht werden, dass mit der magnetischen Korrektur nicht versehentlich mechanische Unwuchten erzeugt werden, was bei einer Korrektur am Rotor zur Steigerung der Laufruhe beiträgt.

Wenn das magnetisch nicht wirksame Material zum Korrigieren einer mechanischen Unwucht des Rotors auf den Rotor aufgebacht wird, kann dieses beispielsweise in Bezug auf eine Massenträgheitsachse des Rotors und in Bezug auf nicht rotationssymmetrisch auf den Rotor aufgebrachtes magnetisch wirksames Material an einer gegenüberliegenden Seite des Rotors aufgebracht werden. Das magnetisch nicht wirksame Material kann beispielsweise im Wesentlichen die gleiche Dichte oder eine ähnliche Dichte aufweisen wie das magnetisch wirksame Material, wodurch die Korrektur der mechanischen Unwucht besonders einfach wird. Beispielsweise kann das magnetisch wirksame Material im Wesentlichen Eisen und das magnetisch nicht wirksame Material im Wesentlichen unmagnetischen, insbesondere antiferromagnetischen, Edelstahl enthalten. In anderen Ausführungen kann es sich bei dem magnetisch wirksamen und dem magnetisch nicht wirksamen Material beispielsweise um unterschiedlich gefüllte Polymere handeln. Unterschiedliche Dichten von magnetisch wirksamem und magnetisch unwirksamem Ausgleichsmaterial können beispielsweise durch gezielt ungleiche Profile der Materialien ausgeglichen werden. Durch das Aufbringen des magnetisch nicht wirksamen Materials wird in der Regel die geometrische Symmetrieachse des Rotors mit seiner Massenträgheitsachse in Übereinstimmung gebracht. Ein Entfernen von magnetisch wirksamem und/oder magnetisch nicht wirksamem Material kann zum Beispiel in präziser Weise mittels Schleifen durchgeführt werden.

In einigen Ausführungen wird die Korrektur der Auslenkung des Rotors durch nicht rotationssymmetrisches Magnetisieren oder Entmagnetisieren von magnetisch wirksamem Material des Rotors durchgeführt. Hierbei wird in der Regel keine lokale Massenveränderung erzeugt. Somit wird auf diese Weise erreicht, dass durch die magnetische Korrektur des Rotors nicht versehentlich eine mechanische Unwucht erzeugt wird. Das Magnetisieren oder Entmagnetisieren kann beispielsweise thermisch und gegebenenfalls unter Anlegen eines Magnetfelds durchgeführt werden. Beispielsweise kann das magnetisch wirksame Material mit einem Laser oder einem Lötkolben lokal über die Curietemperatur des Materials erwärmt werden.

Die vorliegende Anmeldung betrifft auch eine entsprechend vorteilhafte Lageranordnung für eine implantierbare Blutpumpe. Die Lageranordnung kann durch ein wie oben oder unten beschriebenes Verfahren hergestellt sein. Außerdem betrifft die vorliegende Anmeldung eine entsprechend vorteilhafte implantierbare Blutpumpe. Die Blutpumpe kann den Elektromotor mit dem Rotor und dem Stator umfassen. Außerdem kann die Blutpumpe die Lageranordnung mit dem passiven magnetischen Radiallager umfassend den Rotorlagermagneten und ggfs. weitere Rotorlagermagneten und den Statorlagermagneten und ggfs. weitere Statorlagermagneten umfassen. Die Blutpumpe kann außerdem ein auf den Rotor und/oder auf den Stator nicht radialsymmetrisch aufgebrachtes magnetisch wirksames Material zum Definieren und/oder Korrigieren einer radialen Auslenkung des Rotors umfassen. Typischerweise ist die radiale Auslenkung des Rotors derart definiert bzw. korrigiert, dass die geometrische Schwerpunktachse des Rotors mit der Zylindersymmetrieachse des Strömungskanals übereinstimmt oder entsprechend der Kräfteverhältnisse während des Betriebs der Pumpe in eine bestimmte Richtung vorgespannt ist. Das magnetisch wirksame Material kann beispielsweise weich- oder permanentmagnetisch sein. In einigen Ausführungen ist das magnetisch wirksame Material ein beispielsweise weichmagnetischer Streifen, der insbesondere von außen auf einen Teil des Stators aufgebracht ist. In anderen Ausführungen kann das magnetisch wirksame Material ein magnetisch wirksamer Stab, insbesondere ein halbrunder Stab, beispielsweise ein Eisenstab, sein, der an dem Rotor, insbesondere in einer innenliegenden Aussparung des Rotors, vorgesehen ist. Für den Streifen bzw. den Stab ist besonders zuverlässig vorhersehbar, welche Länge und Dicke eine wie starke magnetische Korrektur der Auslenkung hervorruft.

Die implantierbare Blutpumpe kann auch ein magnetisches Axiallager, insbesondere ein passives magnetisches Axiallager umfassen. Durch dieses Lager kann der Rotor in axialer Richtung magnetisch vorgespannt sein, beispielsweise indem der Rotor gegen eine Auflagefläche eines Kontaktlagers, z. B. eines sphärischen oder asphärischen Kalotten- oder Kegellagers, gedrückt wird.

In der Regel wird die Auslenkung des Rotors derart korrigiert, dass eine Spaltbreite zwischen dem Rotor, insbesondere der Beschaufelung, und einer Innenwand des Stators, insbesondere den Rotor vollständig umlaufend, zumindest 20 und/oder höchstens 500 µm beträgt.

Es ist bei dem vorgeschlagenen Verfahren in der Regel so, dass bei der Korrektur ein gewisser Bestandteil des magnetisch aktiven Materials des Rotors bzw. des Stators erhalten bleibt. Insbesondere kann vorgesehen sein, dass bei der Korrektur zumindest 50, insbesondere zumindest 80, Vol.-% des magnetisch aktiven Materials des Stators bzw. Rotors und insbesondere des (bzw. der) Statorlagermagneten bzw. des (bzw. der) Rotorlagermagneten mit unveränderten magnetischen Eigenschaften erhalten bleibt. Somit ist normalerweise beispielsweise kein vollständiger Austausch des Statorlagermagneten bzw. des Rotorlagermagneten vorgesehen.

Oben oder unten in Bezug auf das Herstellungsverfahren beschriebene Merkmale sind entsprechend auf die Lageranordnung bzw. auf die Blutpumpe anwendbar und umgekehrt.

Ausführungsbeispiele werden nachfolgend anhand der Abbildungen beschrieben. Es zeigen
- Fig. 1: eine schematische Ansicht einer Blutpumpe, die in einem Körper eines Patienten implantiert ist,
- Fig. 2: eine schematische Ansicht eines Elektromotors der Blutpumpe,
- Fign. 3(a) bis (c): schematische Ansichten zur Illustration verschiedener Verfahrensschritte bei der Herstellung der Blutpumpe und
- Fig. 4: eine Darstellung zur Illustration einer Korrektur einer Rotorlage des Elektromotors.

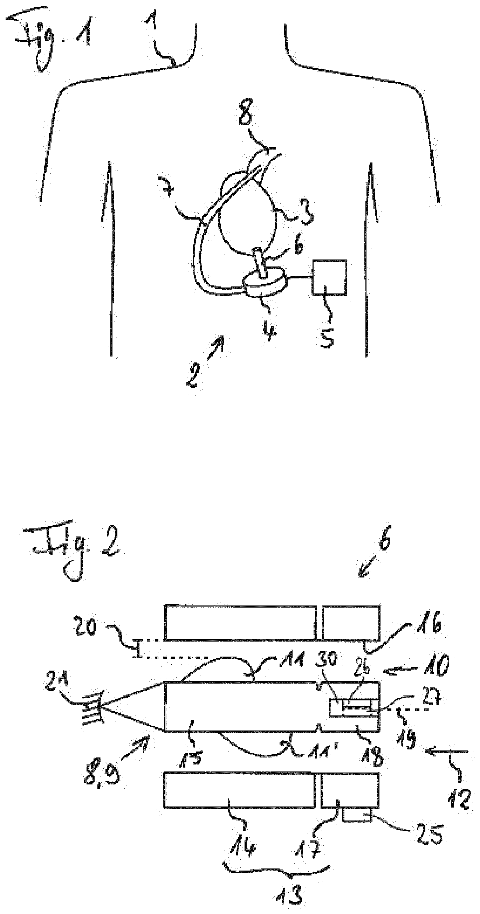

Figur 1 zeigt schematisch einen Körper 1 eines Patienten, in dem eine Blutpumpe 2 zur Unterstützung einer Funktion des Herzens 3 implantiert ist. Die Blutpumpe 2 weist einen typischerweise als Elektromotor mit einem rotierbaren Förderelement ausgeführten Motor auf, der in einem Pumpengehäuse 4 der Blutpumpe 2 aufgenommen ist. Das Pumpengehäuse 4 ist mit einem Steuergerät 5 verbunden, das ebenfalls implantiert sein kann, wie schematisch gezeigt ist. Das Steuergerät 5 kann in einigen Ausführungen vollständig oder teilweise ebenfalls in dem implantierten Pumpengehäuse 4 aufgenommen sein. In anderen Ausführungen ist das Steuergerät 5 extrakorporal angeordnet. Das Pumpengehäuse 4 umfasst außerdem einen mit einer Einlasskanüle des Pumpengehäuses 4 verbundenen Einlasskanal 6, über den Blut aus einer Kammer des Herzens 3 entnommen und über eine Kanüle 7 in ein Blutgefäß 8 gefördert werden kann. Das Steuergerät 5 ist eingerichtet, den Motor der Blutpumpe 2 zum Fördern des Blutes anzusteuern.

Figur 2 zeigt eine schematische Darstellung des Elektromotors 6 der Blutpumpe 2. Wiederkehrende Merkmale sind in dieser Abbildung und in den nachfolgenden Abbildungen mit den gleichen Bezugszeichen versehen. Der Elektromotor 6 ist in dem Pumpengehäuse 4 aufgenommen und umfasst einen Rotor 8 und einen Stator 13. Der Rotor 8 weist das rotierbare Förderelement 9 auf oder bildet dieses. Durch eine in dem dargestellten Bereich im Wesentlichen zylindrische Innenwand 16 des Stators 13 ist ein Strömungskanal 10 begrenzt, durch den beim Betrieb der Blutpumpe 2 Blut gefördert wird. Der Rotor 8 bzw. das Förderelement 9 umfassen eine Beschaufelung 11, 11' zum Fördern des Bluts beispielsweise in einer Richtung, die der Richtung des Pfeils mit dem Bezugszeichen 12 entgegengesetzt ist, und in Richtung der in Fig. 1 gezeigten Kanüle 7. Um eine Rotation des Rotors 8 zum Fördern des Bluts zu erreichen, wird ein von dem Steuergerät 5 elektronisch kontrollierter Stromfluss in Wicklungen 14 des Stators 13 erzeugt. Durch das durch den Stromfluss in den Wicklungen 14 erzeugte Magnetfeld kann ein Antriebsmagnet 15 des Rotors 8, der beispielsweise als Permanentmaget ausgeführt sein kann und der typischerweise starr mit restlichen Bestandteilen des Rotors 8 und insbesondere mit der Beschaufelung 11, 11' verbunden ist, in Drehung versetzt werden.

Der Elektromotor 6 umfasst außerdem eine Lageranordnung mit einem magnetischen Radiallager, das beispielsweise als passives Lager ausgeführt sein und einen oder mehrere Statorlagermagneten 17 und einen oder mehrere Rotorlagermagneten 18 aufweisen kann. Der oder die Statorlagermagnet(e) 17 und der oder die Rotorlagermagnet(e) 18 umfassen jeweils einen oder mehrere Permanentmagneten, durch deren Zusammenwirken eine radiale Lage einer Rotorachse 19, die einer Symmetrieachse des Rotors 8 entspricht, eingestellt wird. In bevorzugten Ausführungen wird die Lage der Rotorachse 19 bei der Herstellung der Blutpumpe 2, wie unten näher erläutert ist, so eingestellt, dass die Rotorachse 19 im Betrieb mit einer Symmetrieachse (Zylinderachse) des Strömungskanals 10 übereinstimmt. Auf diese Weise kann auch ein Abstand 20 oder eine Spaltbreite zwischen der Beschaufelung 11, 11' und der Begrenzung 16 des Strömungskanals 10 präzise eingestellt werden. In bevorzugten Ausführungen beträgt dieser Abstand weniger als 500 µm, beispielsweise 100 µm.

In einigen Ausführungen wird der Rotor 8 durch die gezeigte Vorrichtung auch in axialer Richtung in Position gehalten und somit gelagert. Zu diesem Zweck kann der Elektromotor 6 auch ein Kontaktlager 21 wie beispielsweise ein Spitzenlager oder ein Kalottenlager aufweisen, das eine Bewegung des Rotors 8 in oder entgegengesetzt der Strömungsrichtung 12 begrenzt. Es kann zudem vorgesehen sein, dass der Statorlagermagnet 17 zur axialen Vorspannung des Rotors 8 so auf den Rotorlagermagneten 18 wirkt, dass der Rotor 8 insbesondere in Ruheposition, d.h. ohne dass der Rotor 8 angetrieben wird, gegen das Kontaktlager 21 gedrückt wird. Der Druck des Rotors 8 auf das Kontaktlager 21 kann beim Betrieb der Blutpumpe 2 durch einen Schub des Rotors 8 verringert werden, so dass der Rotor 8 axial reibungsarm gelagert ist. Das weiter unten im Detail beschriebene Verfahren zur Herstellung der Blutpumpe 2 ist bevorzugt auf das hier beschriebene passive magnetische Radiallager anwendbar. Es kann aber ebenso vorteilhaft auf beispielsweise zusätzlich oder alternativ vorgesehene andere Lagertypen, wie beispielsweise Axiallager, angewendet werden. Außerdem lässt sich das Herstellungsverfahren vorteilhaft sowohl für Axialpumpen, als auch für Radialpumpen anwenden.

Wenn Permanentmagnete, wie beispielsweise Ringmagnete, zur Herstellung des Stators 13 oder des Rotors 8 verwendet werden, kann es zu Abweichungen von einer gewünschten von diesen Permanentmagneten hervorgerufenen Magnetfeldverteilung kommen. Solche Fertigungstoleranzen sind insbesondere dann nicht zu vernachlässigen, wenn Blutpumpen 2 mit einer geringen Spaltbreite 20 im Strömungskanal 10 im Bereich des Rotors 8 hergestellt werden sollen. Eine Abweichung der magnetischen Eigenschaften der bei der Herstellung verwendeten Statorlagermagneten 17 kann beispielweise dazu führen, dass eine Symmetrieachse des von dem Statorlagermagneten 17 erzeugten Magnetfeldes gegenüber der geometrischen Symmetrieachse des Strömungskanals 10 radial verschoben ist, also beispielsweise nicht auf der Zylinderachse des Strömungskanals 10 liegt. Zudem kann es beispielsweise bei Abweichungen des Rotorlagermagneten 18 von seinen gewünschten magnetischen Eigenschaften dazu kommen, dass eine Massenträgheitsachse oder eine geometrische Symmetrieachse des Rotors 8 nicht mit seiner magnetischen Schwerpunktachse übereinstimmt.

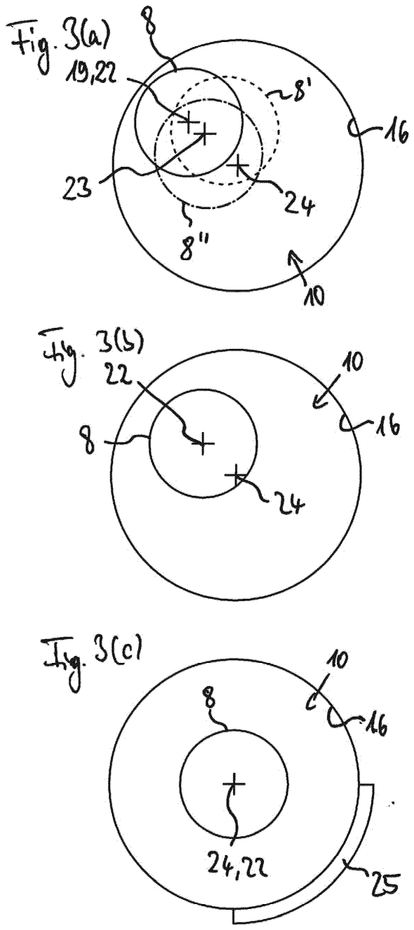

Derartige unerwünschte Abweichungen der magnetischen Eigenschaften der verwendeten Permanentmagnete können bei der Herstellung der Blutpumpe 2 korrigiert werden, wie in Fign. 3(a) bis (c) in schematischen Ansichten in axialer Richtung dargestellt ist. Durch die Innenwand 16 des Stators 13 wird der Strömungskanal 10 begrenzt, in dem der Rotor 8 mit dem Förderelement aufgenommen ist. In dem dargestellten Beispiel stimmt eine Massenträgheitsachse 22 des Rotors 8 mit seiner geometrischen Symmetrieachse 19 überein. Falls die Massenträgheitsachse 22 des Rotors 8 in anderen Ausführungen nicht mit seiner geometrischen Symmetrieachse übereinstimmt, d.h. wenn eine mechanische Unwucht vorliegt, können in an sich bekannter Weise Massenkorrekturen der mechanischen Unwucht vorgenommen werden. Die Massenträgheitsachse 22 des Rotors 8 stimmt allerdings häufig aufgrund der oben beschriebenen Fertigungstoleranzen der verwendeten Permanentmagneten 18 nicht mit einer magnetischen Schwerpunktachse 23 des Rotors 8 überein, was in einer magnetischen Unwucht des Rotors 8 resultiert.

Wenn der Elektromotor 6 der Blutpumpe 2 bei geringen Drehzahlen betrieben wird, kommt die magnetische Unwucht des Rotors 8 zum Tragen, so dass der Rotor 8 nicht um seine Massenträgheitsachse 22, sondern um seine magnetische Schwerpunktachse 23 rotiert. Daraus resultiert eine zeitabhängige Rotorlage in Form einer Taumel-, Schwingungs- bzw. Schlingerbewegung des Rotors 8, bei der sich der Rotor 8 auf einer kreisähnlichen Bahn im Pumpenrohr bewegt, was in Fig. 3(a) durch die Positionen des im dargestellten Beispiel im Uhrzeigersinn taumelnden Rotors 8', 8" zu verschiedenen Zeitpunkten illustriert ist. Wenn die Rotorlage instabil ist, ist eine Auslenkung, d.h. ein Abstand des Rotors 8 von der Innenwand 16 des Stators 13 oder ein Abstand des Rotors 8 von der geometrischen Symmetrieachse 24 des Strömungskanals 10, aufgrund der Taumelbewegung des Rotors 8 zeitabhängig.

Bei der Herstellung des Elektromotors 6 der Blutpumpe 2 kann zunächst der Rotor 8 im durch die Innenwand 16 des Stators 13 begrenzten Strömungskanal 10 angeordnet werden. Der Elektromotor 6 kann anschließend angetrieben werden, beispielsweise unter Bedingungen, die nahezu den Bedingungen entsprechen, die im Betrieb bei implantierter Blutpumpe 2 vorliegen. Zum Beispiel kann bei der Herstellung des Elektromotors 6 zu Testzwecken eine Flüssigkeit durch den Strömungskanal gefördert werden. Die Rotorlage kann während der Herstellung des Elektromotors 6, während die Rotation des Rotors 8 angetrieben wird, beispielsweise durch Beobachtung unter einem Mikroskop oder durch Verwendung von Abstandssensoren überwacht werden.

Anhand einer Beobachtung der Taumelbewegung des Rotors 8 bei geringen Drehzahlen kann eine Korrektur der magnetischen Eigenschaften des Rotorlagermagneten 18 vorgenommen werden, wie unten genauer beschrieben ist. Durch diese Korrektur, die gegebenenfalls mehrere Iterationsschritte umfassen kann, kann die Massenträgheitsachse 22 des Rotors 8 in Übereinstimmung mit der magnetischen Schwerpunktachse 23 des Rotors 8 gebracht werden. Sobald diese Übereinstimmung erreicht ist, rotiert der Rotor 8 bei geringen Drehzahlen um die gemeinsame Massenträgheits- und magnetische Schwerpunktachse 22, 23, wie in Fig. 3(b) illustriert ist.

Die Drehbewegung des Rotors 8 ist mit zunehmender Drehzahl selbststabilisierend, so dass der Rotor 8 bei höheren Drehzahlen um seine Massenträgheitsachse 22 rotiert, auch wenn keine Korrektur der magnetischen Eigenschaften des Rotorlagermagneten 18 vorgenommen wurde, d.h. wenn die Massenträgheitsachse 22 des Rotors 8 nicht mit seiner magnetischen Schwerpunktachse 23 übereinstimmt. Beispielsweise rotiert der Rotor 8 bei einer Nenndrehzahl der Blutpumpe 2, d.h. bei einer Drehzahl, mit welcher der Elektromotor 6 in der Regel betrieben wird, wenn die Blutpumpe 2 zum Fördern von Blut in dem Patienten implantiert ist, selbststabilisiert. In diesem Fall, das heißt für eine selbststabilisierte Rotation des Rotors 8 bei hohen Drehzahlen, ergibt sich somit ebenfalls die in Fig. 3(b) gezeigte Situation, bei der der Rotor 8 mit stabilisierter Rotorlage um seine Massenträgheitsachse 22 rotiert.

Wenn der Rotor 8 mit stabiler Rotorlage rotiert, kann es aufgrund von Fertigungstoleranzen in den magnetischen Eigenschaften des Stators 13 und insbesondere des Statorlagermagneten 17 vorkommen, dass die Rotationsachse des Rotors 8 nicht mit einer gewünschten Rotorlage auf der Symmetrieachse 24 des Strömungskanals 10 übereinstimmt. Somit kann sich bei der stabilen Rotorlage eine unerwünschte, zeitlich konstante Auslenkung der Rotorlage in Bezug auf die Symmetrieachse 24 des Strömungskanals 10 zeigen.

Anhand der Beobachtung der Auslenkung der Rotorlage kann anschließend eine Korrektur der magnetischen Eigenschaften des Stators 13 bzw. des Statorlagermagneten 17 vorgenommen werden, wobei wiederum mehrere Iterationsschritte (Beobachten der Rotorlage mit nachfolgendem Korrigieren der magnetischen Eigenschaften, erneutes Beobachten der Rotorlage usw.) durchgeführt werden können. Insbesondere können die Schritte des Bestimmens der Auslenkung und des Korrigierens wiederholt durchgeführt werden, bis die bestimmte Auslenkung des Rotors 8 einen festgelegten Sollwert unterschreitet. Durch die Korrektur der magnetischen Eigenschaften des Stators 13 kann die Massenträgheitsachse 22, um die der Rotor 8 rotiert, mit der Symmetrieachse 24 des Strömungskanals 10 in Übereinstimmung gebracht werden, wie in Fig. 3(c) gezeigt ist. Auf diese Weise kann gewährleistet werden, dass bei einem Betrieb des Elektromotors 6 der Blutpumpe 2 bei Nenndrehzahl eine Größe des Spalts 20 genau festgelegt wird, was insbesondere bei kleinen Bauformen der Blutpumpe 2 nützlich ist. Die Korrektur der magnetischen Eigenschaften des Stators 13 kann beispielsweise durch Befestigen eines magnetisch wirksamen Materials 25, insbesondere eines ferromagnetischen Materials (bspw. ein Shimming-Eisen oder -Blech) in einem Quadranten des Stators 13 erfolgen, wie in Fig. 3(c) schematisch dargestellt ist. Oben und unten sind jedoch auch weitere Möglichkeiten zur Korrektur der magnetischen Eigenschaften des Stators 13 beschrieben.

Der in der Fig. 2(b) dargestellte Elektromotor 6 ist sowohl in Hinblick auf die magnetischen Eigenschaften des Rotors 8 als auch in Hinblick auf die magnetischen Eigenschaften des Stators 13 korrigiert. Der Rotor 8 weist im Bereich des Rotorlagermagneten 18 und im Bereich seiner zylindrischen Symmetrieachse 19 eine zylindrische Öffnung 30 auf, die beispielsweise zumindest teilweise halbseitig mit einem Stab aus einem magnetisch aktiven Material 26 und halbseitig mit einem Stab aus magnetisch inaktivem Material 27 ausgefüllt ist. Das magnetisch aktive Material 26 umfasst typischerweise ferromagnetisches Material, beispielsweise kann es sich hierbei um einen halbrunden Eisenstab handeln. Durch das magnetisch aktive Material 26 wird die magnetische Schwerpunktachse 23 des Rotors 8 bei der Korrektur der magnetischen Eigenschaften des Rotors 8 mit der Massenträgheitsachse 22 des Rotors 8 in Übereinstimmung gebracht. Das magnetisch inaktive Material 27 ist vorgesehen, damit durch die Korrektur keine nennenswerte mechanische Unwucht erzeugt wird. Das magnetisch inaktive Material 27 kann beispielsweise eine Ausgleichsmasse sein und insbesondere nahezu dieselbe Dichte aufweisen wie das magnetisch aktive Material 26, sodass eine Korrektur der magnetischen Eigenschaften ohne das Erzeugen einer mechanischen Unwucht in vergleichsweise einfacher Weise durchgeführt werden kann. Das magnetisch inaktive Material 26 kann in dem vorliegenden Beispiel als halbrunder Stab aus nicht magnetisierbarem Stahl ausgebildet sein. Es wäre in anderen Ausführungen zusätzlich oder alternativ möglich, dass bei der Korrektur der magnetischen Eigenschaften des Rotors 8 bereits vorhandenes magnetisch aktives Material des Rotorlagermagneten 18 in Teilbereichen eines Umfangs des Rotors 8, d.h. nicht rotationssymmetrisch, entfernt oder hinsichtlich der magnetischen Eigenschaften verändert (magnetisiert oder entmagnetisiert) wird, beispielsweise durch lokales Erhitzen mittels Laser oder Lötkolben.

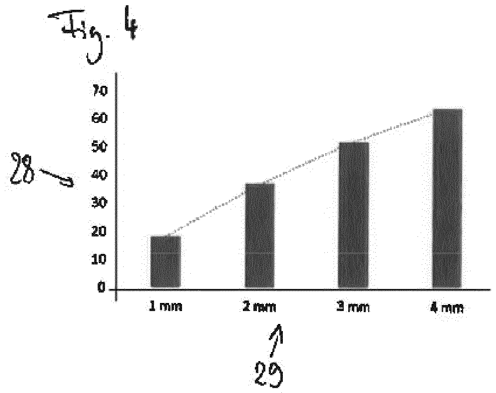

Die Korrektur der magnetischen Eigenschaften des Stators 13 erfolgt in dem dargestellten Beispiel durch Aufbringen des magnetisch aktiven Materials 25 auf eine Außenseite der Statorlagermagneten 17. Figur 4 illustriert, dass eine Korrektur der magnetischen Eigenschaften des Statorlagermagneten 17 sehr effektiv und zielgerichtet erfolgen kann. Aufgetragen ist eine Korrektur 28 der oben beschriebenen statischen Auslenkung der Rotorlage in µm, d.h. eine Verschiebung der Lage der Massenträgheitsachse des Rotors 8 in Richtung der Symmetrieachse 24 des Strömungskanals, über einer axialen Ausdehnung 29 des Shimming-Bleches 25 mit einer Dicke von 200 µm. Mit einer axialen Ausdehnung des Shimming-Bleches 25 von 4 mm lässt sich beispielsweise gezielt eine Korrektur der Rotorlage von etwa 60 µm erzeugen. Es wäre in anderen Ausführungen zur Korrektur der magnetischen Eigenschaften des Stators 13 zusätzlich oder alternativ möglich, dass bereits vorhandenes magnetisch aktives Material des Statorlagermagneten 17 in Teilbereichen eines Umfangs des Stators 13, d.h. nicht rotationssymmetrisch, entfernt oder hinsichtlich der magnetischen Eigenschaften verändert (magnetisiert oder entmagnetisiert) wird, beispielsweise durch Erhitzen mittels Laser oder Lötkolben.

Lediglich in den Ausführungsbeispielen offenbarte Merkmale der verschiedenen Ausführungsformen können miteinander kombiniert und einzeln beansprucht werden.

## Patentansprüche

1. Verfahren zur Herstellung einer Lageranordnung für eine implantierbare Blutpumpe (2), umfassend folgende Schritte:
- Bereitstellen eines Rotors (8) mit einem oder mehreren Antriebsmagneten (15), wobei der Rotor (8) ein Förderelement (9) aufweist,
- Bereitstellen eines Stators (13) mit Statorwindungen,
- Anordnen des Rotors (8) in einem durch eine Innenwandung des Stators (13) gebildeten Strömungskanal (10),
- Antreiben einer Rotordrehung,
- Bestimmen einer Auslenkung des Rotors (8) während die Rotordrehung angetrieben wird,
- Korrigieren der Auslenkung des Rotors (8) durch nicht rotationssymmetrisches Aufbringen, Entfernen, Magnetisieren und/oder Entmagnetisieren von magnetisch wirksamem Material an dem Stator (13) und/oder an dem Rotor (8).

2. Verfahren nach Anspruch 1, **dadurch gekennzeichnet, dass** die Lageranordnung ein magnetisches Radiallager mit einem oder mehreren Rotorlagermagneten (18) und einem oder mehreren Statorlagermagneten (17) umfasst, wobei während des Antriebs der Rotordrehung eine radiale Auslenkung des Rotors (8) bestimmt und anschließend reduziert wird.

3. Verfahren nach Anspruch 2, **dadurch gekennzeichnet, dass** die Bestimmung der radialen Auslenkung bei einem Antrieb der Rotordrehung mit einer Drehzahl durchgeführt wird, bei der der Rotor (8) im Wesentlichen um seine Massenträgheitsachse (22) rotiert, wobei anschließend die radiale Auslenkung des Rotors (8) durch nicht rotationssymmetrisches Aufbringen, Entfernen, Magnetisieren und/oder Entmagnetisieren von magnetisch wirksamem Material (25) an dem Stator (13) reduziert wird.

4. Verfahren nach einem der Ansprüche 2 oder 3, **dadurch gekennzeichnet, dass** die Bestimmung der radialen Auslenkung bei einem Antrieb der Rotordrehung mit einer Drehzahl durchgeführt wird, bei der der Rotor (8) im Wesentlichen um seine magnetische Schwerpunktachse rotiert, wobei anschließend die Auslenkung des Rotors (8) durch nicht rotationssymmetrisches Aufbringen, Entfernen, Magnetisieren und/oder Entmagnetisieren von magnetisch wirksamem Material an dem Rotor (8) korrigiert wird.

5. Verfahren nach einem der Ansprüche 1 bis 4, **dadurch gekennzeichnet, dass** die Auslenkung des Rotors (8) während des Antriebs der Rotordrehung mittels Mikroskop und/oder mittels Abstandssensoren bestimmt wird.

6. Verfahren nach einem der Ansprüche 1 bis 5, **dadurch gekennzeichnet, dass** die Auslenkung des Rotors (8) durch Aufbringen eines magnetisierbaren und/oder magnetisierten Materials (25) korrigiert wird.

7. Verfahren nach einem der Ansprüche 1 bis 6, **dadurch gekennzeichnet, dass** die Korrektur der Auslenkung des Rotors (8) durch nicht rotationssymmetrisches Aufbringen oder Entfernen von magnetisch wirksamem Material (26) an dem Rotor (8) durchgeführt wird und dass zusätzlich magnetisch nicht wirksames Material (27) zum Korrigieren einer mechanischen Unwucht des Rotors (8) vom Rotor (8) entfernt bzw. auf den Rotor (8) aufgebacht wird.

8. Verfahren nach einem der Ansprüche 1 bis 7, **dadurch gekennzeichnet, dass** die Korrektur der Auslenkung des Rotors (8) durch nicht rotationssymmetrisches Magnetisieren oder Entmagnetisieren von magnetisch wirksamem Material des Rotors (8) durchgeführt wird.

9. Lageranordnung für eine implantierbare Blutpumpe (2), hergestellt durch ein Verfahren nach einem der Ansprüche 1 bis 8.

10. Implantierbare Blutpumpe (2), umfassend einen Elektromotor (6) mit einem Rotor (8), einem Stator (13) und einer Lageranordnung mit einem passiven magnetischen Radiallager, umfassend mindestens einen Rotorlagermagneten (18) und mindestens einen Statorlagermagneten (17), **gekennzeichnet durch** ein auf den Rotor (8) und/oder auf den Stator (13) nicht radialsymmetrisch aufgebrachtes magnetisch wirksames Material zum Definieren und/oder Korrigieren einer radialen Auslenkung des Rotors (8).

11. Implantierbare Blutpumpe (2) nach Anspruch 10, **dadurch gekennzeichnet, dass** die radiale Auslenkung des Rotors (8) derart definiert bzw. korrigiert ist, dass eine geometrische Schwerpunktachse des Rotors (8) mit einer Zylindersymmetrieachse eines durch eine Innenwandung des Stators (13) gebildeten Strömungskanals (10) übereinstimmt.
